(19) **Europäisches Patentamt European Patent Office Office européen des brevets**

(11) **EP 4 745 569 A1**

(12) **EUROPÄISCHE PATENTANMELDUNG**

(43) Veröffentlichungstag:
**20.05.2026 Patentblatt 2026/21**

(21) Anmeldenummer: **25214535.4**

(22) Anmeldetag: **10.11.2025**

(51) Internationale Patentklassifikation (IPC):
***G01N 33/00*** *(2006.01)* ***G01N 27/404*** *(2006.01)*

(52) Gemeinsame Patentklassifikation (CPC):
**G01N 33/0006; G01N 33/005;** G01N 27/404

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**GE KH LA MA MD TN**

(30) Priorität: **14.11.2024 DE 102024133368**

(71) Anmelder: **Dräger Safety AG & Co. KGaA 23560 Lübeck (DE)**

(72) Erfinder:
• **Veelken, Henrik 23558 Lübeck (DE)**
• **Eckhardt, Rolf 23558 Lübeck (DE)**
• **Tschuncky, Peter 23558 Lübeck (DE)**
• **Reier, Tobias 23558 Lübeck (DE)**
• **Oertel, Marcus 23558 Lübeck (DE)**

(54) **SYSTEM UND VERFAHREN ZUR KALIBRIERUNG UND BESTIMMUNG EINER KONZENTRATION EINES ANALYTEN IN EINEM TRÄGERGAS EINES QUASIBINÄREN ODER BINÄREN GASGEMISCHS**

(57) System und Verfahren zur Kalibrierung und Bestimmung einer Konzentration eines Analyten in einem Trägergas eines quasibinären oder binären Gasgemischs. Das System umfasst einen elektrochemischen Gassensor, wobei der elektrochemische Gassensor eine Arbeitselektrode und eine Gegenelektrode aufweist, wobei der elektrochemische Gassensor mittels der Arbeitselektrode eingerichtet ist, den Analyten zu oxidieren und/oder zu reduzieren und wenigstens eine Komponente des Trägergases zu reduzieren und/oder zu oxidieren und ein entsprechendes Messsignal bereitzustellen. Das System umfasst ferner eine Speichereinheit, in welcher eine Kalibrierinformation für das Gasgemisch hinterlegt ist, und eine Datenverarbeitungseinheit, welche eingerichtet ist: das Messsignal zu empfangen, die Kalibrierinformation zu empfangen, die Konzentration des Analyten in dem Trägergas aus dem Messsignal und aus der Kalibrierinformation zu bestimmen, und die bestimmte Konzentration des Analyten in dem Trägergas bereitzustellen.

Fig. 2

EP 4 745 569 A1

**Beschreibung**

Die vorliegende Erfindung betrifft ein System und Verfahren zur Bestimmung einer Konzentration eines Analyten in einem Trägergas eines quasibinären oder binären Gasgemischs.

Systeme und Verfahren zur Bestimmung einer Konzentration eines Analyten in einem Trägergas eines quasibinären oder binären Gasgemischs sind grundsätzlich bekannt.

Im Hinblick auf die zunehmende Wichtigkeit von Alternativen zu fossilen Brennstoffen rückt die Wasserstoffproduktion in den Fokus. In diesem Zusammenhang werden Elektrolyseure verwendet, um aus Wasser ($H_2O$) Sauerstoff ($O_2$) und Wasserstoff ($H_2$) zu erzeugen. Bei den Elektrolyseuren muss an den Elektroden die ordnungsgemäße Funktion der Elektrolysezellen überwacht werden. Dazu wird im Kathodengas, das hauptsächlich Wasserstoff enthält, überwacht, dass nur wenig oder kein Sauerstoff anwesend ist, um eine Fehlfunktion des Elektrolyseurs oder auch die Entstehung eines explosionsfähigen Knallgasgemisches zu vermeiden, was insbesondere bei höheren Drücken auf der Anodenseite als auf Kathodenseite eintreten kann. Dabei treten naturgemäß hohe $H_2$-Konzentrationen (90 bis 100 Vol.%) auf. Eine explosionsfähige Gasmischung tritt bei etwa 95% $H_2$ und 5% $O_2$ auf. Dabei ist das Gasgemisch im stationären Zustand ein binäres Gasgemisch. Dessen Zusammensetzung ist zu überwachen, ohne dem Prozess eine Zündquelle beizusteuern.

Demnach besteht die Notwendigkeit, die Konzentration des erzeugten Sauerstoffs in dem erzeugten Wasserstoff und/oder die Konzentration des erzeugten Wasserstoffs in dem erzeugten Sauerstoff auf einfache Weise bestimmen zu können. Hierbei handelt es sich also jeweils beispielsweise um ein binäres Gasgemisch. Es kann in diesem Zusammenhang auch erforderlich sein, die Konzentration von Sauerstoff oder Wasserstoff in einem anderen Trägergas, welches eine Gasmischung wie Luft sein, zu ermitteln. Bei diesem Beispiel handelt es sich dann also um ein quasibinäres Gasgemisch.

DE 20 2009 003 553 U1 beschreibt insofern eine Möglichkeit, Gase zu identifizieren und Gasmengen sowie die Zusammensetzungen binärer Gasmischungen experimentell zu bestimmen. Dieses gilt auch für niedrig siedende Flüssigkeiten und binäre Flüssigkeitsgemische bei einem Druck unterhalb des Sättigungsdampfdrucks. Hierzu wird ein akustisches Resonanzrohr mit dem entsprechenden Gas, der binären Gasmischung oder mit einer niedrig siedende Flüssigkeiten oder einem binären Flüssigkeitsgemisch gefüllt und stehende Wellen mit einem Lautsprecher im Bereich zwischen 0 bis einigen kHz erzeugt. Die Resonanzfrequenz, bei der sich die stehenden Wellen herausbilden, ist abhängig von der Gasart, der Art der Gasmischung bzw. der niedrig siedende Flüssigkeiten oder des binären Flüssigkeitsgemisches, von der Temperatur, der Länge des Resonanzrohrs und von dem Gesamtdruck.

Ferner ist für die Analyse von binären oder quasibinären Gasgemischen die Verwendung von Sensoren, welche eine Wärmeleitfähigkeit des Gasgemisches untersuchen, bekannt. Ein Beispiel eines solchen Sensors ist der Sensor CALOMAT 7 der Firma Siemens. Das Messprinzip dieses Gerätes ist die Messung der Wärmeleitfähigkeit des binären Gasgemisches. So ist die Wärmeleitfähigkeit von $H_2$ 0,1805 $W \cdot m^{-1} \cdot K^{-1}$ und von $O_2$ 0,02658 $W \cdot m^{-1} \cdot K^{-1}$. Mit der sich ergebenden Wärmeleitfähigkeit des Gasgemisches, kann z.B. näherungsweise linear oder mit der Mischungsformel von Mason und Saxena das Mischungsverhältnis von $O_2$ und $H_2$ bestimmt werden. Beide Wärmeleitfähigkeiten sind in diesem Fall positiv und die Wärmeleitfähigkeit des Gemisches liegt zwischen den Wärmeleitfähigkeiten der Einzelkomponenten.

Der vorliegenden Erfindung liegt die Aufgabe zu Grunde, ein alternatives System und entsprechendes Verfahren zur Bestimmung einer Konzentration eines Analyten in einem Trägergas eines quasibinären oder binären Gasgemischs bereitzustellen, insbesondere ein System und entsprechendes Verfahren mit verbesserter Empfindlichkeit.

Diese Aufgabe wird gelöst durch das System nach Anspruch 1 sowie durch das Verfahren nach Anspruch 6.

Die abhängigen Ansprüche, die Figuren und die Beschreibung stellen weitere vorteilhafte Ausgestaltungen der Erfindung bereit.

Erfindungsgemäß wird ein System zur Bestimmung einer Konzentration eines Analyten in einem Trägergas eines quasibinären oder binären Gasgemischs bereitgestellt. Das System umfasst einen elektrochemischen Gassensor, wobei der elektrochemische Gassensor eine Arbeitselektrode und eine Gegenelektrode aufweist, wobei der elektrochemische Gassensor mittels der Arbeitselektrode eingerichtet ist, den Analyten zu oxidieren und/oder zu reduzieren und wenigstens eine Komponente des Trägergases zu reduzieren und/oder zu oxidieren und ein entsprechendes Messsignal bereitzustellen. Das System umfasst ferner eine Speichereinheit, in welcher eine Kalibrierinformation für das Gasgemisch hinterlegt ist, und eine Datenverarbeitungseinheit, welche eingerichtet ist: das Messsignal zu empfangen, die Kalibrierinformation zu empfangen, die Konzentration des Analyten in dem Trägergas aus dem Messsignal und aus der Kalibrierinformation zu bestimmen, und die bestimmte Konzentration des Analyten in dem Trägergas bereitzustellen.

Auf diese Weise ist es möglich, mittels eines elektrochemischen Gassensors, welcher an der Arbeitselektrode sowohl den Analyten aus auch wenigstens teilweise das Trägergas umsetzt und lediglich unter Verwendung einer neuartig erzeugten Kalibrierinformation für ein quasibinäres oder binäres Gasgemisch eine Konzentration des Analyten in dem Trägergas des quasibinären oder binären Gasgemischs zu bestimmen. Dies ermöglicht insbesondere die Verwendung bekannter elektrochemischer Gassensoren und reduziert somit den Aufwand der Bereitstellung des Systems.

Darüber hinaus kann die gleichzeitige Umsetzung von Analyt und wenigstens eines Teils des Trägergases zu einer Verstärkung des Messsignals führen, wodurch die Empfindlichkeit des System verbessert werden kann. Dies tritt dann ein, wenn der Analyt reduktiv oder oxidativ und die wenigstens eine Komponente des Trägergases komplementär oxidativ oder reduktiv an der Arbeitselektrode umgesetzt werden.

Unter einem Gasgemisch wird ein Gemisch aus Gasen verstanden, nämlich eine Mischung aus dem zu untersuchenden Analyten mit dem Trägergas. Ein binäres Gasgemisch ist ein Gemisch aus dem Analyt mit einem reinen Gas als Trägergas, wie beispielsweise eine Mischung von $O_2$ als Analyt und $H_2$ als Trägergas oder eine Mischung von $H_2$ als Analyt und $O_2$ als Trägergas. Ein quasibinäres Gasgemisch ist ein Gemisch aus dem Analyt mit einem Gasgemisch als Trägergas, wie beispielsweise eine Mischung von $O_2$ als Analyt und Luft als Trägergas oder eine Mischung von $H_2$ als Analyt und Luft als Trägergas, bei dem wenigstens eine Komponente des Trägergases an der Arbeitselektrode umgesetzt werden kann und die übrigen Komponenten des Trägergases an der Arbeitselektrode (bei dem gleichen Arbeitspotential) elektrochemisch inert sind. Bei einem quasibinären Gasgemisch muss erfindungsgemäß die Zusammensetzung des Trägergases, beispielsweise in Form einer vorbestimmten Information, bekannt sein.

Der elektrochemische Gassensor kann auf bekannte Weise ausgestaltet sein und kann neben der Arbeitselektrode und Gegenelektrode optional weitere Elektroden, wie eine weitere Arbeitselektrode und/oder eine weitere Gegenelektrode und/oder eine Referenzelektrode umfassen.

Das Messsignal kann beispielweise ein Messstrom oder eine Messspannung sein oder ein mit einem Messstrom oder einer Messspannung korrelierendes Signal.

Die Kalibrierinformation kann beispielsweise als eine look-up-Tabelle, als eine analytisch auszuwertende Funktion oder als eine sonstige Information ausgestaltet sein, welche eine Kalibrierkurve indiziert.

Die Bestimmung der Konzentration des Analyten in dem Trägergas aus dem Messsignal und aus der Kalibrierinformation kann beispielsweise durch (ggf. direkten) Vergleich des Messsignals mit der Kalibrierinformation erfolgen. Weitere Schritte der Signalverarbeitung und/oder der Datenverarbeitung können optional durchgeführt werden, z.B. zur Aufbereitung des Messsignals oder um aus dem Messsignal erhältliche zwischenverarbeitete Werte zu erhalten, welche zum Vergleich mit der Kalibrierinformation geeignet sind.

Die Datenverarbeitungseinheit kann beispielsweise als Komponente eines Gasmessgeräts ausgestaltet sein, welches den elektrochemischen Gassensor aufweisen kann oder zur Aufnahme des elektrochemischen Gassensors ausgestaltet sein.

In einem weiteren Beispiel kann die Datenverarbeitungseinheit als handgehaltenes Gerät, wie als Smartphone oder als Computer ausgestaltet sein und eingerichtet sein, das Messsignal kabelgebunden oder kabellos zu erhalten.

Die Speichereinheit kann beispielsweise als Komponente eines Gasmessgeräts, eines handgehaltenen Geräts, wie eines Smartphones oder als Komponente eines Computers ausgestaltet sein.

Das Bereitstellen des Messsignals kann über eine datentechnische Schnittstelle zur weiteren Datenverarbeitung erfolgen und/oder als Ausgabe über eine Mensch-Maschine-Schnittstelle wie ein Display erfolgen.

Das Bereitstellen des Messsignals kann umfassen, einen Alarmierungszustand abhängig von der bestimmten Konzentration des Analyten in Relation zu einer kritischen Konzentration des Analyten zu bestimmen und bei Bestimmen, dass der Alarmierungszustand vorliegt, eine Ausgabe eines Alarms über eine Alarmierungseinrichtung wie eine Sirene durchzuführen.

Vorzugsweise indiziert die Kalibrierinformation eine Kalibrierkurve in einem Koordinatensystem, wobei in dem Koordinatensystem die Konzentration des Analyten oder eine mit der Konzentration des Analyten korrespondierende Größe eine Abszisse bildet und das Messsignal oder eine mit dem Messsignal korrespondierende Größe eine Ordinate bildet, wobei die Kalibrierkurve nicht durch einen Koordinatenursprung des Koordinatensystems verläuft.

Dies stellt eine rechentechnisch besonders einfach Möglichkeit dar, die Kalibrierinformation bereitzustellen.

Beispielsweise kann der Versatz der Kalibrierkurve von dem Koordinatenursprung mind. +- 50 µA bis +- 800 µA betragen. Ob der Versatz positiv oder negativ ausgestaltet ist, ist abhängig davon, ob der Analyt an der Arbeitselektrode oxidiert oder reduziert wird.

Vorzugsweise ist die Kalibrierkurve als Kalibriergerade mit einem positiven Ordinaten-Achsenabschnitt und mit einer negativen Steigung ausgestaltet.

Alternativ vorzugsweise ist die Kalibrierkurve als Kalibriergerade mit einem negativen Ordinaten-Achsenabschnitt und mit einer positiven Steigung ausgestaltet.

Vorzugsweist ist der Analyt Sauerstoff oder Wasserstoff, wobei das Trägergas komplementär dazu Wasserstoff oder Sauerstoff ist oder wobei das Trägergas komplementär dazu Wasserstoff oder Sauerstoff umfasst.

Vorzugsweise weist der elektrochemische Gassensor ferner eine Referenzelektrode und eine Schutzelektrode auf.

Auf diese Weise kann verhindert werden, dass nicht an der Arbeitselektrode umgesetztes Gas in den Gassensor eindringt und dort ein Referenzpotential der Referenzelektrode verschiebt.

Erfindungsgemäß wird ferner ein Verfahren zur Bestimmung einer Konzentration eines Analyten in einem Trägergas eines quasibinären oder binären Gasgemischs bereitgestellt. Das Verfahren weist die Schritte auf: Empfangen eines Messsignals, wobei das Messsignal mittels Oxidation und/oder Reduktion des Analyten und Reduktion und/oder Oxidation wenigstens einer Komponente des Trägergases an einer gemeinsamen Arbeitselektrode erhältlich ist, Empfangen einer Kalibrierinformation für das Gasgemisch, Bestimmen der Konzentration des Analyten in dem Trägergas aus dem Messsignal und aus der Kalibrierinformation, und Bereitstellen der bestimmten Konzentration des Analyten in dem Trägergas.

Das Verfahren weist zu dem System vergleichbare vorteilhafte Wirkungen auf. Alle im Zusammenhang mit dem System offenbarten Merkmale gelten auch als im Zusammenhang mit dem Verfahren offenbart und andersherum.

Das Verfahren ist insbesondere ein computerimplementiertes Verfahren.

Vorzugsweise ist die gemeinsame Arbeitselektrode die Arbeitselektrode des beschriebenen Gassensors und die Datenverarbeitungseinheit des Systems ausgestaltet, einige oder alle Schritte des Verfahrens durchzuführen.

Diese und weitere Vorteile und Merkmale der Erfindung ergeben sich auch aus der nachfolgenden Figurenbeschreibung. Dabei zeigt:

**Fig. 1**    ein Ausführungsbeispiel eines erfindungsgemäßen Systems,

**Fig. 2**    Ausführungsbeispiele von Kalibrierkurven,

**Fig. 3**    ein beispielhafter Verlauf eines Messignals über die Zeit, das mit dem erfindungsgemäßen System nach Fig. 1 erhältlich ist, und

**Fig. 4**    ein Ausführungsbeispiel eines erfindungsgemäßen Verfahrens, das mittels des Systems nach Fig. 1 ausführbar ist.

Erfindungsgemäß wird ein System 1000 zur Bestimmung einer Konzentration c eines Analyten in einem Trägergas eines quasibinären oder binären Gasgemischs G bereitgestellt.

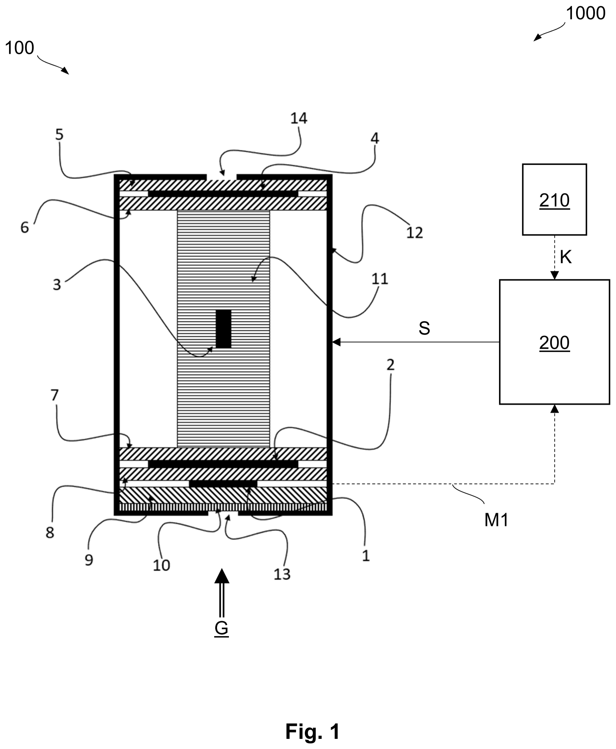

Ein Ausführungsbeispiel des erfindungsgemäßen Systems 1000 ist in Fig. 1 dargestellt. Das System 1000 umfasst einen elektrochemischen Gassensor 100, wobei der elektrochemische Gassensor 100 zumindest eine Arbeitselektrode 1 und eine Gegenelektrode 4 aufweist. Der elektrochemische Gassensor 100 ist mittels der Arbeitselektrode 1 eingerichtet, den Analyten zu oxidieren und/oder zu reduzieren und wenigstens eine Komponente des Trägergases (komplementär) zu reduzieren und/oder zu oxidieren und ein entsprechendes Messsignal M1 bereitzustellen. Das System 1000 weist ferner eine Speichereinheit 210 auf, in welcher eine Kalibrierinformation K für das Gasgemisch G hinterlegt ist. Das System 1000 weist außerdem eine Datenverarbeitungseinheit 200 auf, welche eingerichtet ist, das Messsignal M1 zu empfangen, die Kalibrierinformation K zu empfangen, die Konzentration c des Analyten in dem Trägergas aus dem Messsignal M1 und aus der Kalibrierinformation K zu bestimmen, und die bestimmte Konzentration C des Analyten in dem Trägergas bereitzustellen.

Die Datenverarbeitungseinheit 200 kann eingerichtet sein, mittels eines Signals S einen Betrieb des elektrochemischen Gassensors 100 zu steuern.

Der elektrochemische Gassensor 100 kann weitere Komponenten aufweisen.

Wie in Fig. 1 dargestellt ist, kann der elektrochemische Gassensor 100 ein Gehäuse 12 aufweisen, das einige oder alle Komponenten des elektrochemische Gassensor 100 aufnimmt.

Ein Gaseintritt des Gasgemisches G in den elektrochemische Gassensor 100 kann durch eine Gehäuseöffnung 13 und eine Membran 10 erfolgen. Das Material der Membran 10 ist vorzugsweise ein Polymer aus Perfluoalkoxy (PFA), Polytetrafluorethylen (PTFE), Perfluorethylenpropylen (FEP), Polyvinylidenfluorid (PVDF), Polyethylen (PE) oder aus Polypropylen (PP). Die Dicke des Materials kann zwischen 1 $\mu$m und 100 $\mu$m liegen. Die Membran 10 ist vorzugsweise flüssigkeitsdicht und gasdurchlässig und kann als Stoffflussbegrenzungsmembran fungieren.

In Gaseintrittsrichtung (vgl. Pfeil das Gasgemisches G in Fig. 1) betrachtet hinter der Membran 10 kann optional eine weitere Stützmembran 9 angeordnet sein. Sie kann dazu dienen, Membran 10 mechanisch zu stützen, da diese typischerweise sehr dünn ausgeführt ist. Das Material der Stützmembran 9 umfasst zum Beispiel poröses PTFE, PE oder PP. Die Membran 9 kann außerdem als Träger der Arbeitselektrode 1 ausgestaltet sein. Es ist auch möglich, die Membran 10 dicker auszugestalten und auf den Einsatz der Stützmembran 9 zu verzichten.

Die Arbeitselektrode 1 kann als eine Platinmetall-haltige Elektrode und bevorzugt als eine Sinterelektrode ausgestaltet sein. An der Arbeitselektrode 1 findet die Sensorreaktion statt. Beispielweise wird, wenn der Analyt Sauerstoff ist und das Trägergas Wasserstoff ist, an der Arbeitselektrode 1, abhängig vom Elektrodenpotential, Sauerstoff reduziert und Wasserstoff oxidiert, beispielsweise gemäß nachstehender Elektrodengleichung:

$$O_2 + 4e^- + 4H^+ \quad \rightarrow \quad 2\ H_2O$$
$$H_2 \quad \rightarrow \quad 2\ H^+ + 2e^-$$

Das aus dem Messstrom erhältliche Messsignal M1 kann zur Ermittlung beispielsweise des Sauerstoffgehaltes in dem Wasserstoff herangezogen werden.

Es wird somit erfindungsgemäß sowohl der Analyt als auch gleichzeitig das Trägergas bzw. eine Komponente des Trägergases an der Arbeitselektrode 1 elektrochemisch umgesetzt. Der Stromfluss (Messstrom) der Arbeitselektrode 1 und somit das Messsignal M1 ergibt sich somit als Summe der Stromflüsse des Analyten und des Trägergas bzw. der Komponente des Trägergases. Die Beiträge der Einzelströme aus Analyten-Umsetzung und Trägergas-Umsetzung können dabei ein gleiches oder ein verschiedenes Vorzeichen aufweisen. Die Gewichtung der Beiträge wird durch die jeweilige Kalibrierinformation K abgebildet. Die Kalibrierinformation K berücksichtigt den Umsatz beider Gase (Analyt und Trägergas) und dient zur Umrechnung des Stromflusses bzw. des Messsignals M1 in die Konzentration c des Analyten. Bei einem binären oder quasibinären Gasgemisch können dabei beide Gehalte, Analyt-Anteil und Trägergas-Anteil, bestimmt werden.

Der erfindungsgemäße elektrochemische Gassensor 100 weist im Betrieb zum einen den Vorteil auf, dass das Vorhandensein des Analyten in dem binären oder quasibinären Gasgemisch G einen entsprechenden Anteil an Trägergas verdrängt und dadurch das Signal des Trägergases verringert. Zum anderen steigt, abhängig von Analyt und Trägergas, mit zunehmendem Analyt-Anteil der dem Analyt-Anteil entsprechende reduktive oder oxidative Anteil des Messstroms, der aus der Analyt-Umsetzung an der Arbeitselektrode 1 entsteht. Der elektrochemische Gassensor 100 reagiert somit aufgrund dieser sich gegenseitig verstärkenden Effekte, sehr sensitiv auf auftretenden Analyten. Gegenüber einer Verdrängung eines Gases durch ein anderes Gas führt dieser gegenläufige Reaktionsverlauf zu einer relativen Verstärkung des Signals, wenn der Analyt Sauerstoff oder Wasserstoff ist, und das Trägergas komplementär dazu Wasserstoff oder Sauerstoff ist oder das Trägergas komplementär dazu Wasserstoff oder Sauerstoff umfasst, verglichen mit einer Gasmischung aus z.B. Wasserstoff und Stickstoff, bei dem der Stickstoff nicht umgesetzt würde, sondern nur über seine Verdrängung das Wasserstoff-Signal erniedrigen würde.

Das erfindungsgemäße System 1000 weist somit einen deutlich einfacheren Aufbau auf als herkömmliche Systeme, die auf einer anderen Sensortechnologie basieren. Ferner weist das resultierende Messsignal, das mit dem erfindungsgemäßen System 1000 erhältlich ist, auf Grund der gegenläufigen Reaktionsmechanismen für eine Messung von O2 in H2 oder H2 in O2 eine Verstärkung auf, so dass die Empfindlichkeit des Systems 1000 verbessert ist.

In Gaseintrittsrichtung betrachtet hinter der die Arbeitselektrode 1 kann optional eine weitere Membran 8 angeordnet sein. Die Membran 8 kann zwei Funktionen erfüllen. Zum einen kann die Membran 8 eine Benetzung der Arbeitselektrode 1 sowie der noch zu beschreibenden optionalen Schutzelektrode 2 mit Elektrolyten sicherstellen, um diese in einem elektrochemischen Halbzellensystem miteinander zu verbinden. Zum anderen verhindert Membran 8 elektrische Kurzschlüsse zwischen der Arbeitselektrode 1 und der optionalen Schutzelektrode 2. Die Membran 8 kann beispielsweise als Vlies aus mineralischen und/oder aus organischen Fasern bereitgestellt sein. Als besonders geeignet haben sich Vliese aus Glasfasern oder Polyolefin erwiesen.

Optional kann der elektrochemischen Gassensor 100 eine Schutzelektrode 2 aufweisen. Die Schutzelektrode 2 kann bevorzugt aus dem gleichen Material wie die Arbeitselektrode 1 bereitgestellt sein. Ferner bevorzugt kann die Schutzelektrode 2 im gleichen Potentialbereich, ganz besonders bevorzugt bei einem identischen Potential wie die Arbeitselektrode 1 betrieben werden. Es ist dargestellt und bevorzugt, dass die Schutzelektrode 2 eine größere Fläche aufweist als die Arbeitselektrode 1. Die Schutzelektrode 2 dient dazu, nicht an der Arbeitselektrode 1 umgesetztes Gas, z.B. H2 oder O2, umzusetzen und so zu verhindern, dass diese Gase in den Sensorrückraum eindringen und dort das Referenzpotential der optionalen Referenzelektrode 3 verschieben. Gegenüber einem 3-Elektroden-Sensor kann so ein dauerhaft stabiles Messverhalten erzielt werden. Insbesondere wenn Wasserstoff als Teil des binären Gasgemisches vorliegt, besteht das Problem, dass Wasserstoff permeabler ist als andere Gase und dadurch leicht in den elektrochemischen Gassensor 100 eindringen kann, ohne von der Arbeitselektrode 1 vollständig verbraucht zu werden. Dies kann eine Anreicherung des Wasserstoffs im Inneren des elektrochemischen Gassensor 100 zur Folge haben und zu einem instabilen Messsignal führen.

In Gaseintrittsrichtung betrachtet hinter der optionalen Schutzelektrode 2 kann der elektrochemische Gassensor 100 ferner eine weitere Membranen 6 und 7 aufweisen. Diese Membranen 6 und 7 können funktions- und ggf. auch materialgleich mit Membran 8 ausgestaltet sein.

Zwischen den Membranen 6 und 7 kann eine weitere Membran 11 angeordnet sein, welche als Stützkernelement fungieren und somit einen mechanischen Kontakt der Komponenten des elektrochemischen Gassensor bewirken kann. Die weitere Membran 11 kann ferner als Docht für den Elektrolyten fungieren. Der Stützkern 11 kann beispielsweise als ein Glassinterkörper oder als eine Kunststoffhülse mit einem Elektrolyt-saugenden Kern ausgestaltet sein.

Der elektrochemische Gassensor 100 kann ferner optional eine Referenzelektrode 3 aufweisen.

Die Gegenelektrode 4 kann beispielsweise als eine Sinterelektrode aus einem Edelmetall, bevorzugt aus Platin, bereitgestellt sein. An der Gegenelektrode 4 läuft in Summe eine zur Arbeitselektrode 1 entgegengesetzte Reaktion ab, wodurch die Elektroneutralität der Gesamtzelle (des elektrochemischen Gassensors 100) gewährleistet wird. Mögliche Reaktionen an der Gegenelektrode 4 sind:

| O2 + 4e- + 4H+ | --> | 2H2O |
|---|---|---|
| 2H+ + 2e- | --> | H2, |
| 2H2O | --> | O2 + 4e- + 4H+ |

Der elektrochemische Gassensor 100 kann ferner optional eine als Druckausgleichsventil fungierende weitere Membran 5 aufweisen. Die weitere Membran 5 kann in oder an einer Gehäuseöffnung 14 des elektrochemischen Gassensors 100 angeordnet sein. Die weitere Membran 5 kann aus porösem PTFE bereitgestellt sein. Die weitere Membran 5 kann zum einen die Gegenelektrode 4 tragen und zum anderen dafür sorgen, dass an der Gegenelektrode 4 gebildete Gase den elektrochemische Gassensor 100 verlassen können. Darüber hinaus kann die weitere Membran 5 einen Druckausgleich durch die Gehäuseöffnung 14 des elektrochemische Gassensor 100 bereitstellen.

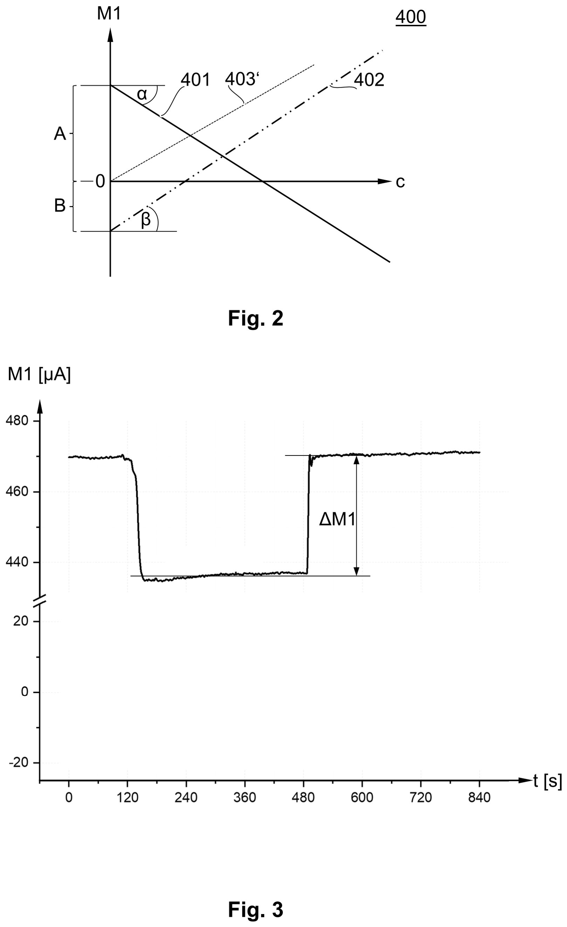

In Fig. 2 sind drei Kalibriergeraden 401, 402, 403' als beispielhafte Ausgestaltungen von Kalibrierinformationen K in einem Koordinatensystem 400 dargestellt. Die Kalibriergeraden 401, 402 sind dabei erfindungsgemäß, während die Kalibriergeraden 403' eine nicht erfindungsgemäße Kalibriergerade eines herkömmlichen elektrochemischen Gassensors aus dem Stand der Technik darstellt.

Es ist aus Fig. 2 ersichtlich, dass die Kalibrierinformation K somit eine Kalibrierkurve 401, 402, z.B. eine Kalibriergerade 401, 402, in einem Koordinatensystem 400 indizieren kann, wobei in dem Koordinatensystem 400 die Konzentration des Analyten c oder eine mit der Konzentration des Analyten c korrespondierende Größe eine Abszisse bildet und das Messsignal M1 oder eine mit dem Messsignal M1 korrespondierende Größe eine Ordinate bildet, wobei die Kalibrierkurve 401, 402 nicht durch einen Koordinatenursprung 0 des Koordinatensystems 400 verläuft.

Abhängig davon, ob der Analyt oxidativ oder reduktiv an der Arbeitselektrode 1 umgesetzt wird, kann die Kalibrierkurve 401 als Kalibriergerade 401 mit einem positiven Ordinaten-Achsenabschnitt A und mit einer negativen Steigung $\alpha$ ausgestaltet sein, oder die Kalibrierkurve 402 kann als Kalibriergerade 402 mit einem negativen Ordinaten-Achsenabschnitt B und mit einer positiven Steigung $\beta$ ausgestaltet sein.

Die Parameter A, $\alpha$, B und $\beta$ sind mittels einer Kalibrierung erhältlich und können als Teil der Kalibrierinformation K in der Speichereinheit 210 hinterlegt werden.

Die Kalibrierung kann beispielsweise mittels nachfolgendem Ablauf erfolgen. Der elektrochemische Gassensor 100 kann mit dem Arbeitspotential vorgespannt und mit 100 Vol% Trägergas (z.B. H2) begast werden. Sobald ein Sättigungswert erreicht ist, wird dieser Wert mit 0 Vol% Analyt (z.B. O2) kalibriert. Vgl. hierzu Fig. 2. Es wird deutlich, dass wenn c = 0 ist, M1 >> 0 (wenn der Analyt z.B. O2 ist) ist bzw. M1 << 0 (wenn der Analyt z.B. H2 ist und das Trägergas z.B. O2 ist) ist. Danach kann ein definiertes binäres oder quasibinäres Gasgemisch G erstellt werden, z.B. mit 96 Vol% H2 als Trägergas und 4 Vol% O2 als Analyt. Aufgrund der Verdrängung des H2 und des folglich geringeren Sensorstroms und der Reduktion von O2 an der Arbeitselektrode 1 (negativer Strom) ergibt sich dann ein Sensorstrom für 4 Vol% O2 wie folgt:

$$I_{4Vol\% \, O2} = I_{96 \, Vol\% \, H2} + I_{4 \, Vol\% \, O2}$$

$$I_0 - I_{4Vol\% \, O2} = I_{100Vol\% \, H2} - I_{96 \, Vol\% \, H2} - |I_{4 \, Vol\% \, O2}|$$

$$\text{mit } I_{100Vol\% \, H2} > I_{96Vol\% \, H2} \gg 0 \wedge I_{4Vol\% \, O2} < 0$$

In Fig. 3 ist ein beispielhafter erfindungsgemäßer Verlauf eines Messsignals M1 über die Zeit t dargestellt, welches unter Verwendung eines erfindungsgemäßen Systems 1000 erhalten wurde. Im gezeigten Beispiel wurde das System 1000 zunächst mit 100 Vol% Trägergas, nämlich mit H2 begast. Das resultierende Messsignal M1 liegt bei ca. 470 $\mu$A. Etwa zum Zeitpunkt t = 120 s wurde dem System 1000 ein Gasgemisch G aus Trägergas und Analyt zugeführt. Dies führte zu einem Signalabfall $\Delta M$ 1 von ca. 35$\mu$A. Durch die Kalibrierinformation K konnte bestimmt werden, dass das veränderte Messsignal M1 von ca. 435 $\mu$A einer Konzentration des Analyten in dem Trägergas von ca. 4 % entspricht.

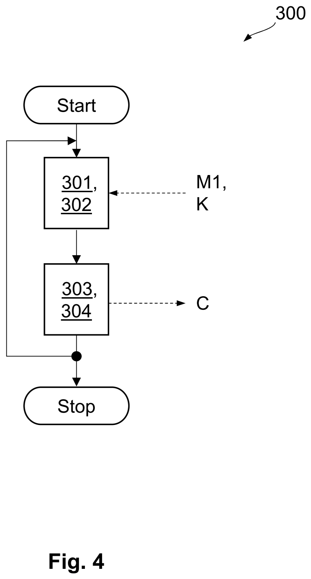

In Fig. 4 ist ein Ausführungsbeispiel eines erfindungsgemäßen Verfahrens 300 zur Bestimmung einer Konzentration c eines Analyten in einem Trägergas eines quasibinären oder binären Gasgemischs G dargestellt.

Das Verfahren 300 weist den Schritt 301: Empfangen eines Messsignals M1, wobei das Messsignal M1 mittels Oxidation und/oder Reduktion des Analyten und Reduktion und/oder Oxidation wenigstens einer Komponente des Trägergases einer gemeinsamen Arbeitselektrode 1 erhältlich ist auf.

Das Verfahren 300 weist den Schritt 302: Empfangen einer Kalibrierinformation K für das Gasgemisch G auf.

Das Verfahren 300 weist den Schritt 303: Bestimmen der Konzentration c des Analyten in dem Trägergas aus dem Messsignal M1 und aus der Kalibrierinformation K auf.

Das Verfahren 300 weist den Schritt 304: Bereitstellen der bestimmten Konzentration C des Analyten in dem Trägergas auf.

Aller hierin offenbarten Merkmale sind beliebig miteinander kombinierbar, sofern dies nicht widersprüchlich ist oder Alternativen betrifft.

**Bezugszeichenliste**

| | |
|---|---|
| 1 | Arbeitselektrode |
| 2 | Schutzelektrode |
| 3 | Referenzelektrode |
| 4 | Gegenelektrode |
| 5 | Membran |
| 6 | Membran |
| 7 | Membran, Separatorvlies |
| 8 | Membran, Separatorvlies |
| 9 | Membran, Stützmembran |
| 10 | Membran, Diffusionsbegrenzer |
| 11 | Membran, Stützkern |
| 12 | Gehäuse |
| 13 | Gaseintrittsöffnung |
| 14 | Druckausgleichsöffnung |
| 100 | Gassensor |
| 200 | Datenverarbeitungseinheit |
| 210 | Speichereinheit |
| 300 | Verfahren |
| 301, 302, ... | Schritte des Verfahrens |
| 400 | Koordinatensystem |
| 401, 402 | Kalibrierkurve |
| 403' | Kalibrierkurve gemäß Stand der Technik |
| 1000 | System |

| | |
|---|---|
| A | erster Achsenabschnitt |
| $\alpha$ | erste Steigung |
| B | zweiter Achsenabschnitt |
| $\beta$ | zweite Steigung |
| c | Konzentration des Analyten |
| C | bestimmte Konzentration des Analyten |
| G | Gas, Gasgemisch |
| K | Kalibrierinformation |
| M1 | Messsignal |
| S | Signal |

**Patentansprüche**

**1.** System (1000) zur Bestimmung einer Konzentration (c) eines Analyten in einem Trägergas eines quasibinären oder binären Gasgemischs (G), umfassend:

- einen elektrochemischen Gassensor (100),

wobei der elektrochemische Gassensor (100) eine Arbeitselektrode (1) und eine Gegenelektrode (4) aufweist,
wobei der elektrochemische Gassensor (100) mittels der Arbeitselektrode (1) eingerichtet ist, den Analyten zu oxidieren und/oder zu reduzieren und wenigstens eine Komponente des Trägergases zu reduzieren und/oder zu oxidieren und ein entsprechendes Messsignal (M1) bereitzustellen,

- eine Speichereinheit (210), in welcher eine Kalibrierinformation (K) für das Gasgemisch (G) hinterlegt ist, und
- eine Datenverarbeitungseinheit (200), welche eingerichtet ist:

- das Messsignal (M1) zu empfangen,
- die Kalibrierinformation (K) zu empfangen,
- die Konzentration (c) des Analyten in dem Trägergas aus dem Messsignal (M1) und aus der Kalibrierinformation (K) zu bestimmen, und
- die bestimmte Konzentration (C) des Analyten in dem Trägergas bereitzustellen.

2. System (1000) nach Anspruch 1,

wobei die Kalibrierinformation (K) eine Kalibrierkurve (401, 402) in einem Koordinatensystem (400) indiziert, wobei in dem Koordinatensystem (400) die Konzentration des Analyten (c) oder eine mit der Konzentration des Analyten (c) korrespondierende Größe eine Abszisse bildet und das Messsignal (M1) oder eine mit dem Messsignal (M1) korrespondierende Größe eine Ordinate bildet, und wobei die Kalibrierkurve (401, 402) nicht durch einen Koordinatenursprung (0) des Koordinatensystems (400) verläuft.

3. System (1000) nach Anspruch 2,

wobei die Kalibrierkurve (401, 402) als Kalibriergerade (401, 402) mit einem positiven Ordinaten-Achsenabschnitt (A) und mit einer negativen Steigung ($\alpha$) ausgestaltet ist, oder wobei die Kalibrierkurve (401, 402) als Kalibriergerade (401, 402) mit einem negativen Ordinaten-Achsenabschnitt (B) und mit einer positiven Steigung ($\beta$) ausgestaltet ist.

4. System (1000) nach einem der vorherigen Ansprüche,

wobei der Analyt Sauerstoff oder Wasserstoff ist, und wobei das Trägergas komplementär dazu Wasserstoff oder Sauerstoff ist oder wobei das Trägergas komplementär dazu Wasserstoff oder Sauerstoff umfasst.

5. System (1000) nach einem der vorherigen Ansprüche, wobei der elektrochemische Gassensor (100) ferner eine Schutzelektrode (2) und eine Referenzelektrode (3) aufweist.

6. Verfahren (300) zur Bestimmung einer Konzentration (c) eines Analyten in einem Trägergas eines quasibinären oder binären Gasgemischs (G), aufweisend die Schritte (301, 302, ...):

- (301) Empfangen eines Messsignals (M1), wobei das Messsignal (M1) mittels Oxidation und/oder Reduktion des Analyten und Reduktion und/oder Oxidation wenigstens einer Komponente des Trägergases einer gemeinsamen Arbeitselektrode (1) erhältlich ist,
- (302) Empfangen einer Kalibrierinformation (K) für das Gasgemisch (G),
- (303) Bestimmen der Konzentration (c) des Analyten in dem Trägergas aus dem Messsignal (M1) und aus der Kalibrierinformation (K), und
- (304) Bereitstellen der bestimmten Konzentration (C) des Analyten in dem Trägergas.

7. Verfahren (300) nach Anspruch 6,

wobei die gemeinsame Arbeitselektrode (1) die Arbeitselektrode (1) des Gassensors (100) nach einem der Ansprüche 1 bis 5 ist, und wobei die Datenverarbeitungseinheit (200) des Systems (1000) nach einem der Ansprüche 1 bis 5 ausgestaltet ist, einige oder alle Schritte des Verfahrens (300) durchzuführen.

**Fig. 1**

**Fig. 2**

**Fig. 3**

300

**Fig. 4**

## EUROPÄISCHER RECHERCHENBERICHT

Europäisches Patentamt
European Patent Office
Office européen des brevets

Nummer der Anmeldung

EP 25 21 4535

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2022 133531 A1 (MSA EUROPE GMBH [CH]) 20. Juni 2024 (2024-06-20) | 1,4,6,7 | INV. $G01N33/00$ |
| Y | * Fig. 1,4,-7 and corresponding text passages * <br> * [0024,0034,0053,0058] * <br> ----- | 5 | ADD. $G01N27/404$ |
| Y | DE 198 45 318 A1 (DRAEGER SICHERHEITSTECH GMBH [DE]) 20. April 2000 (2000-04-20) * Anspruch 8 * <br> ----- | 5 | |
| X | KARBACH NIKLAS ET AL: "Preparation of low concentration H 2 test gas mixtures in ambient air for calibration of H 2 sensors", EGUSPHERE PREPRINT REPOSITRY, 27. März 2024 (2024-03-27), XP093364821, DOI: 10.5194/egusphere-2024-611 * das ganze Dokument * <br> ----- | 1-4,6,7 | |

**RECHERCHIERTE SACHGEBIETE (IPC)**

G01N

Der vorliegende Recherchenbericht wurde für alle Patentansprüche erstellt

| Recherchenort | Abschlußdatum der Recherche | Prüfer |
|---|---|---|
| München | 11. Februar 2026 | Klein, Marc-Oliver |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 25 21 4535

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

11-02-2026

| Im Recherchenbericht angeführtes Patentdokument | Datum der Veröffentlichung | Mitglied(er) der Patentfamilie | Datum der Veröffentlichung |
|---|---|---|---|
| DE 102022133531 A1 | 20-06-2024 | AU 2023397898 A1 | 29-05-2025 |
| | | CA 3268745 A1 | 20-06-2024 |
| | | CN 120077267 A | 30-05-2025 |
| | | DE 102022133531 A1 | 20-06-2024 |
| | | EP 4634654 A1 | 22-10-2025 |
| | | JP 2025541058 A | 18-12-2025 |
| | | WO 2024126008 A1 | 20-06-2024 |
| DE 19845318 A1 | 20-04-2000 | DE 19845318 A1 | 20-04-2000 |
| | | FR 2784190 A1 | 07-04-2000 |
| | | GB 2342168 A | 05-04-2000 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 202009003553 U1